(19) Europäisches Patentamt
European Patent Office
Office européen des brevets

(11) **EP 1 645 229 B1**

(12) **EUROPÄISCHE PATENTSCHRIFT**

(45) Veröffentlichungstag und Bekanntmachung des Hinweises auf die Patenterteilung:
**26.11.2008 Patentblatt 2008/48**

(51) Int Cl.:
*A61B 5/103* (2006.01)   *A61B 17/02* (2006.01)
*A61F 2/46* (2006.01)   *A61B 19/00* (2006.01)

(21) Anmeldenummer: **04024165.5**

(22) Anmeldetag: **11.10.2004**

(54) **Bänderkraft-Erfassungssystem**

System for determining the ligament force

Système pour déterminer la force des ligaments

(84) Benannte Vertragsstaaten:
**DE FR GB IT**

(43) Veröffentlichungstag der Anmeldung:
**12.04.2006 Patentblatt 2006/15**

(73) Patentinhaber: **BrainLAB AG**
**85551 Kirchheim/Heimstetten (DE)**

(72) Erfinder:
• **Neubauer, Timo**
  **85622 Feldkirchen (DE)**
• **Plassky, Norman**
  **99099 Erfurt (DE)**
• **Wagner, Benjamin**
  **81669 München (DE)**

(74) Vertreter: **Schwabe - Sandmair - Marx**
**Patentanwälte**
**Stuntzstrasse 16**
**81677 München (DE)**

(56) Entgegenhaltungen:
WO-A-20/04041097    DE-A- 10 221 274
US-A- 4 501 266    US-A- 5 630 820

• **C. MARMIGNON, A. LEMNIEI, P. CINQUIN AND A. HODGSON: "A COMPUTER-ASSISTED CONTROLLED DISTRACTION DEVICE TO GUIDE LIGAMENT BALANCING DURING KNEE ARTHROPLASTY" PROCEEDINGS OF THE FOURTH ANNUAL CONFERENCE OF THE INTERNATIONAL SOCIETY FOR COMPUTER ASSISTED ORTHOPAEDIC SURGERY CAOS, 19. Juni 2004 (2004-06-19), XP008043736 CHICAGO USA**

**Beschreibung**

Die Erfindung betrifft ein Bänderkraft-Erfassungssystem. Solche Systeme dienen auf dem medizinischen Gebiet dazu, Körperstrukturen, meist Knochenstrukturen auf Spannung auseinander zu halten und die dabei auftretenden Kräfte an den Bändern fest zu stellen. Die entsprechenden Kräfte sollen abgeglichen werden, wenn z. B. Bandstrukturen manipuliert werden, um die anatomische Lage der Beinachse wiederherzustellen. Dies gilt speziell für Fälle, in denen Gelenkprothesen eingesetzt werden.

Im US-Patent Nummer 5,470,354 und in der europäischen Patenanmeldung EP 1 402 857 A2 sind Kraft- bzw. Spannungssensoren beschrieben, wobei eine provisorische Tibia-Komponente mit einem eingebauten Druck/Kraft-Sensor verwendet wird, um die Bänderspannung interoperativ während des Setzens einer Kniegelenkprothese zu messen.

Solche Systeme haben den Nachteil, dass die Sensoren in dem Einsatz, also dem Teil, das zwischen die Knochenstruktur eingesetzt wird, angeordnet sind und ihre Signale, das heißt die Kraft- oder Spannungsdaten übermitteln müssen, was meist per Leitung geschieht. Ferner müssen noch Energieversorgungen bereitgestellt werden, und all diese Gegebenheiten machen herkömmliche Systeme kompliziert, schwierig zu handhaben, relativ teuer und schwer zu sterilisieren.

Aus der WO 2004/041097 A1 ist ein Verfahren und eine Vorrichtung zur Bestimmung der Lage einer Kniegelenkendoprothese bekannt, bei dem unter Einsatz eines Navigationssystems der distale Femur und der proximale Tibiakopf bei gestreckten und bei angewinkelten Knie lateral und medial mittels eines Distraktionsgeräts mit einer definierten Kraft in eine gespreizte Position gebracht werden und dabei jeweils die Relativpositionen von Femur und Tibia und damit die Größe des Spaltes zwischen Femur und Tibia bestimmt werden. In Abhängigkeit davon werden virtuell Anlageflächen bestimmt, an denen die Prothesenteile an Femur und Tibia angelegt werden können. Dieses Verfahren dient beispielsweise zur Bestimmung von Sägeebenen zur Bearbeitung der Knochen.

Aus der US 4,501,266 ist eine Kniespreizvorrichtung bekannt, welche mit zwei Paaren federbelasteter Spreizschuhe ausgestattet ist, welche jeweils in einen Spalt zwischen zwei Gelenkknochen einbringbar sind. Die Vorrichtung ist geeignet, eine Balance und eine gewisse Ausrichtung zwischen den zu spreizenden Knochen zueinander herzustellen.

Aus C. Marmignon, A. Lemniei, P. Cinquin and A. Hodgson: "A computer-assisted controlled distraction device to guide ligament balancing during knee arthroplasty" proceedings of the fourth annual conference of the international society for Computer assisted orthopaedic surgery caos, 19. Juni 2004, XP008043736 Chicago, USA ist eine Vorrichtung bekannt, mit der eine Spreizung von Knochenspalten möglich ist. Die Vorrichtung basiert auf einem hydraulischen Antrieb.

Aus der US 5,630,820 ist ebenfalls eine Vorrichtung zur Ausbalancierung von Ligamenten während einer Kniegelenksprothesenimplantationsoperation bekannt. Die Vorrichtung weist zwei Paare von Spreizschuhen auf, die bei der Betätigung der Vorrichtung parallel zueinander aufgespreizt werden können.

Aus der DE 102 21 274 A1 ist eine Vorrichtung zur Messung der Bandspannung bei der Implantation einer Kniegelenkendoprothese bekannt. Die Vorrichtung weist parallel zueinander verlaufend im Abstand voneinander angeordnete Führungszylinder auf, in welchen jeweils ein, mit einer definierten Federkraft beaufschlagter Führungsschuh zur Aufnahme der Femurkondylen angeordnet ist. Die Bandspannung wird über das Mass der Auslängung des jeweiligen Führungsschuhes gegen die Federkraft gemessen.

Es ist die Aufgabe der vorliegenden Erfindung, ein Bänderkraft-Erfassungssystem bereit zu stellen, welches die genannten Probleme zumindest teilweise, insbesondere vollständig überwindet. Insbesondere soll die Handhabung des Systems und die Kommunikation der Kraft- bzw. Spannungsmessdaten verbessert werden. Insbesondere soll sichergestellt werden, dass die Vorrichtung bei Knieanwendungen benutzt werden kann und seine Funktion erfüllt, wenn die Patella in ihrer anatomischen Position verbleibt. Weiterhin soll der ungehinderte Lauf der Patella erreicht werden

Diese Aufgabe wird erfindungsgemäß durch ein Bänderkraft-Erfassungssystem gemäß dem Anspruch 1 gelöst. Die Unteransprüche definieren vorteilhafte Ausführungsformen der vorliegenden Erfindung.

Ein erfindungsgemäßes Bänderkraft-Erfassungssystem weist einen Einsatz auf, der zwischen zwei durch Bänder miteinander verbundene Körperstrukturen einbringbar ist. Ferner weist es eine Messanordnung auf, welche die Kräfte bestimmt, die auf die Bänder wirken, und in soweit entspricht das erfindungsgemäße System ganz allgemein gesprochen noch denjenigen, die aus dem Stand der Technik bekannt sind. Weiterhin besitzt der Einsatz eine vorbestimmte Elastizität (im Falle einer Feder deren Federkonstante). An den Körperstrukturen sind Referenzanordnungen angeordnet, und die Messanordnung umfasst ein medizinisches Navigationssystem, welches Verschiebungen der Körperstrukturen über die Referenzstrukturen erfasst, so dass aus den Verschiebungen und der bekannten Elastizität auf die Kräfte geschlossen werden kann, welche auf die Bänder wirken.

Gekennzeichnet ist das Bänderkraft-Erfassungssystem gemäß der Erfindung dadurch, dass der Einsatz Außerkörper mit Federn und nebeneinander angeordneten Spreizfortsatzpaaren aufweist, wobei die Außenkörper im Abstand verstellbar miteinander verbunden sind und wobei die Spreizfortsatzpaare hinsichtlich ihrer Länge und Abwinklung eine unterschiedliche Form besitzen.

Mit anderen Worten wird gemäß der Erfindung nicht mehr nur kontinuierlich direkt zwischen den Körperstrukturen (Knochenenden) die Kraft gemessen, um den Kraftwert dann weiter zu kommunizieren, sondern es wird durch die vorbestimmte Elastizität des Einsatzes eine Elastizität bzw. Federkonstante definiert, und die Kraft selbst kann dann einfach dadurch ermittelt werden, dass die entstehenden Verschiebungen bzw. Wege gemessen werden. Solche Wege lassen sich aber "von Außen" durch einfache Messvorgänge (z.B. mit Hilfe medizinischer Navigationssysteme) ermitteln, so dass es nicht mehr notwendig ist, Kraftwerte "aus dem Inneren", nämlich direkt aus dem Einsatz per Draht oder Leitung dorthin zu kommunizieren, wo sie verarbeitet oder genutzt werden.

Damit stellt die Erfindung eine Möglichkeit zur Verfügung, die Kraft- bzw. Spannungsdaten ligamentärer Strukturen in kabelloser Weise und ohne die Verwendung von Wandlern und Sensoren, intraoperativ festzustellen. Der Einsatz kann deshalb als einfaches mechanisches Bauteil ausgestaltet werden und lässt sich ohne Weiteres sterilisieren. Energieversorgungen im Einsatz oder für den Einsatz sind nicht mehr notwendig.

Dadurch, dass ein Spreizfortsatzpaar mit einer bestimmten Form (z.B. länger als das andere) ausgestaltet ist, kann der Einsatz beispielsweise bei Knieanwendungen benutzt werden und seine Funktion auch dann erfüllen, wenn die Patella in ihrer anatomischen Position verbleibt. Diese spezielle Form eines Spreizfortsatzpaares ist eine Voraussetzung für den ungehinderten Lauf der Patella.

Besonders vorteilhaft wirkt sich aus, dass in letzter Zeit immer mehr computerassistierte Kniegelenkersatzoperationen durchgeführt und somit immer mehr Operationsräume mit Navigationssystemen ausgestattet werden. Das erfindungsgemäße System kann in Kombination mit diesen sinnvoll eingesetzt werden.

Gemäß einer Ausführungsform der Erfindung kann der Einsatz eine Feder mit bekannter Federkonstante aufweisen. Die Länge der Feder ist vorteilhafterweise durch einen Stellmechanismus veränderbar, um die Vorspannkraft der Feder in einer Ausgangsstellung festlegen zu können.

Der Einsatz kann so konstruiert werden, dass er einen Außenkörper aufweist, in dem die Feder gelagert ist, und mindestens ein Spreizfortsatzpaar, insbesondere ein Spreizplattenpaar, dass durch die Federn vorgespannt wird und welches zwischen die Körperstrukturen einbringbar ist. Dabei besteht die vorteilhafte Möglichkeit, einen Einsatz zu konstruieren, der zwei solche Außenkörper mit Federn und Spreizfortsatzpaaren aufweist, die nebeneinander angeordnet sind. Im Kniebereich können die Ober- und Unterschenkelknochen dann an beiden Seiten zwischen den Gelenkfortsätzen auseinander gedrückt werden.

Wie oben schon erwähnt besteht eine bevorzugte Ausführungsform des erfindungsgemäßen Systems darin, dass das Navigationssystem aus den Verschiebungen und der Elastizität die Kräfte errechnet, welche auf die Bänder wirken, und diese Informationen in Echtzeit ausgibt.

Eine Ausführungsform der vorliegenden Erfindung wird im Weiteren anhand der beiliegenden Zeichnungen näher erläutert. Die Erfindung kann alle hierin beschriebenen Merkmale einzeln oder in jeder Kombination aufweisen. In den Zeichnungen zeigen:

Figur 1     einen Schnitt durch einen Einsatz eines erfindungsgemäßen Bänderkraft-Erfassungssystems, der im Weiteren auch Bänderbalancevorrichtung genannt wird;

Figur 2     eine perspektivische Ansicht einer aufgeschnittenen Bänderbalancevorrichtung;

Figur 3     einen Schnitt durch einen Teil der Vorrichtung mit Kennzeichnung des Federweges;

Figur 4     ein erfindungsgemäßes System in einer Anwendung an einem gestrecktem Kniegelenk;

Figur 5     einen Screenshot eines Navigationssystems für den Fall der Figur 4;

Figur 6     ein System wie in Figur 4 mit abgewinkeltem Kniegelenk;

Figur 7     einen Screenshot des Navigationssystems für den Fall der Figur 6.

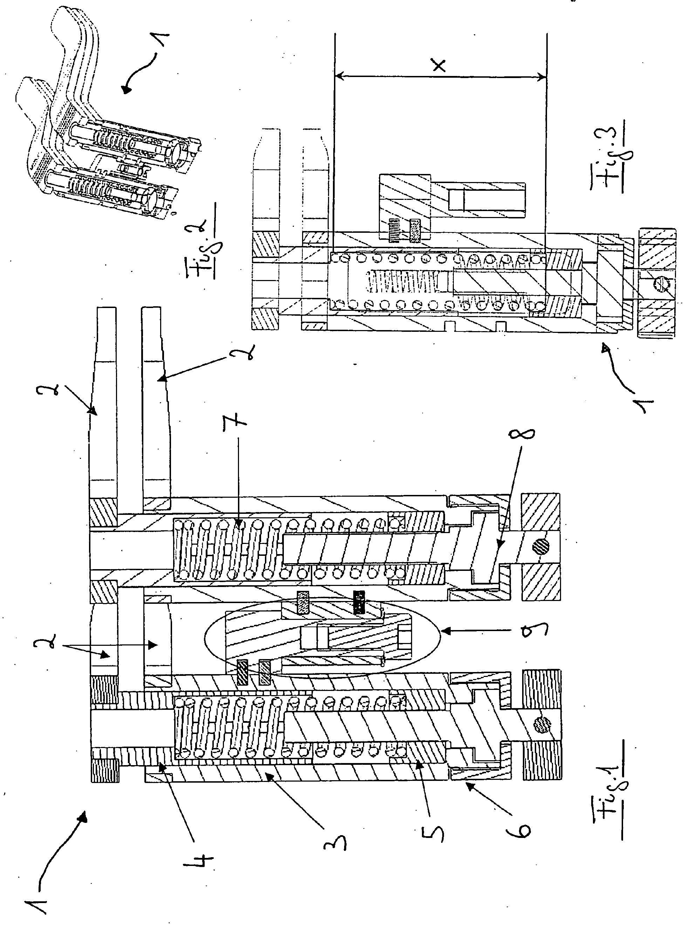

In der Figur 1 ist ein Einsatz eines erfindungsgemäßen Bänderkraft-Erfassungssystems mit dem Bezugszeichen 1 versehen und in einer Schnittdarstellung gezeigt. Dieser Einsatz wird wie oben schon angesprochen, im Weiteren auch als Bänderbalancevorrichtung bezeichnet (Ligament Balancing Device).

Die Vorrichtung 1 weist einen Außenkörper oder eine Außenhülse 3 auf, welche den darin liegenden Mechanismus umgibt. In der Außenhülse 3 ist eine obere Innenhülse 4 geführt, ebenso eine untere Innenhülse 5. Zwischen den beiden Innenhülsen 4,5 ist eine Feder 7 eingesetzt, und diese ist in dem rechten Teil der Vorrichtung 1 nummeriert, der im Wesentlichen ebenso - ausgestaltet ist wie der linke Teil. - Unterhalb der Innenhülse 5 und der Außenhülse 3 ist ein Bodenteil 6 vorgesehen, das an der Außenhülse 3 angeschraubt ist. Dieses Bodenteil 6 sichert eine Rädelschraube 8, welche durch Verdrehung über ein Schraubengetriebe die Feder 7 in ihrer Länge verstellen kann, indem die Innenhülse 5 nach oben und geführt in der Außenhülse 3 angehoben wird. Über die obere Innenhülse 4 sind am rechten und linken Teilstück der Vorrichtung 1 jeweils ein paar Spreizplatten 2 angeordnet, je zwei mediale und laterale Spreizplatten. Die oberen der dargestellten Spreizplatten sind jeweils an der oberen Innenhülse 4 fixiert, die unteren an der Außenhülse 3.

Die Spreizplatten 2 werden durch die Kraft der Feder 7 auseinander gedrückt.

Zwischen dem linken und dem rechten Teil der Vorrichtung 1 liegt eine Gelenkanordnung 9. Mit dieser Gelenkanordnung lassen sich diese beiden Teile gelenkig zueinander verstellen, wodurch sich insgesamt der Abstand der Spreizplatten einstellen lässt. Wie der perspektivischen und geschnittenen Ansicht der Figur 2 entnehmbar ist, weist eines der Spreizplattenpaare eine andere Form auf als das andere. Mit Hilfe des Gelenkmechanismus 9 kann dann die Gesamtvorrichtung 1 so eingestellt werden, dass die beiden Teile nahe aneinander liegen und die Spreizplatten im Wesentlichen in die gleiche Richtung abstehen. Weil eines der Spreizplattenpaare eine bestimmte Form aufweist (Länge, Abwinklung), kann es dann in einen weiter entfernten Zwischenraum eingreifen, und die Vorrichtung 1 insgesamt kann beispielsweise bei Knieanwendungen so positioniert werden, dass die Kniescheibe in ihrer anatomischen Bahn nicht behindert/beeinflusst wird.

Die Figur 3 stellt nur einen Teil der Vorrichtung 1 dar, und sie bezeichnet den Federweg X, auf den später noch Bezug genommen wird.

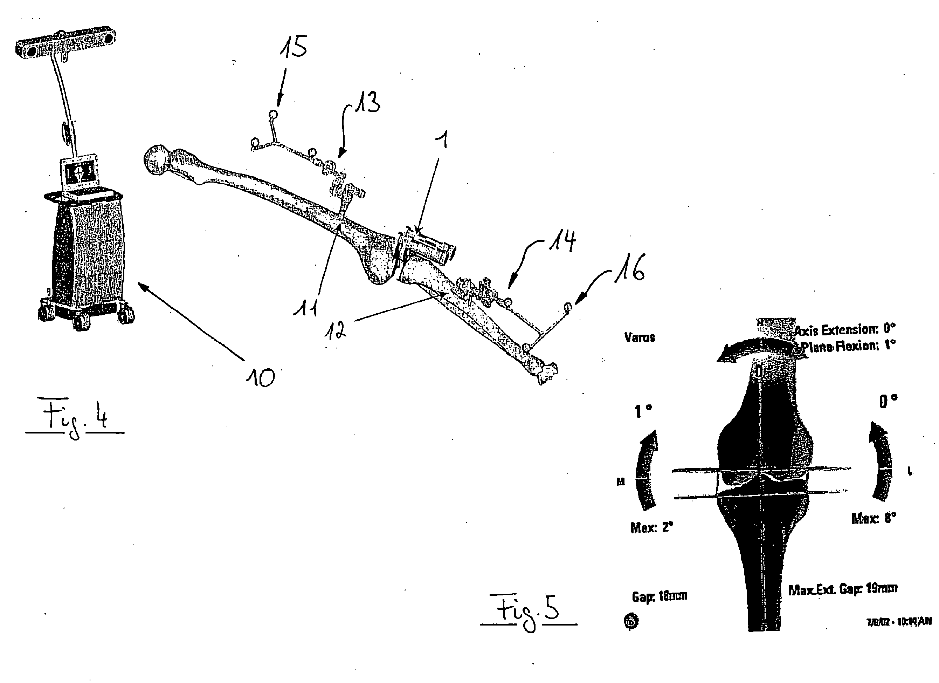

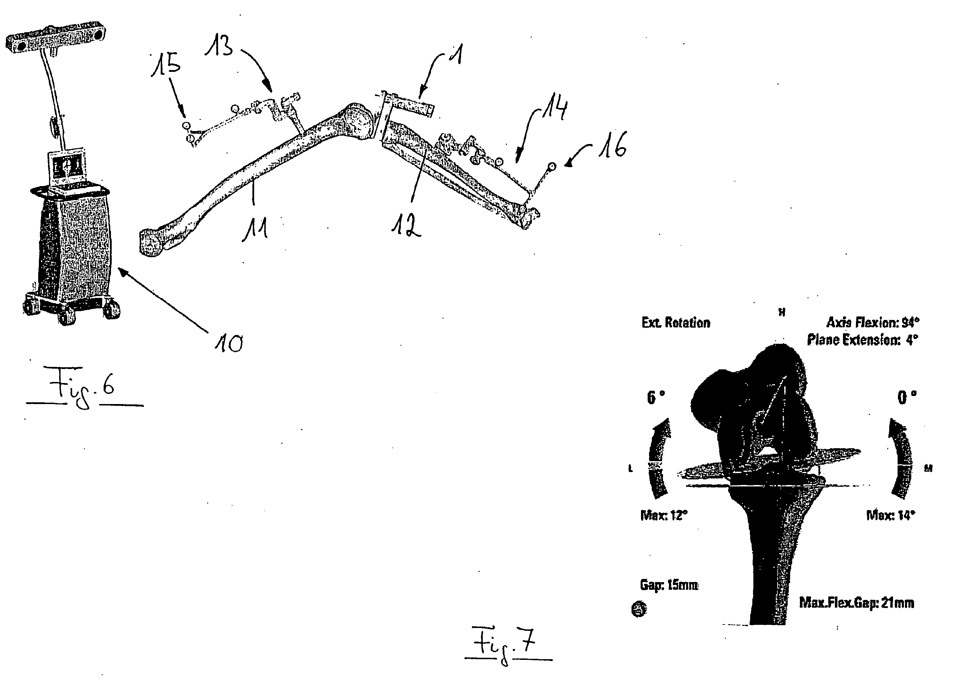

In den Figuren 4 und 6 sind erfindungsgemäße Bänderkraft-Erfassungssysteme in ihrer Gesamtheit dargestellt. Gleiche Bezugszeichen bezeichnen gleiche Teile, der Unterschied liegt lediglich darin, dass der Zustand der Figur 4 eine nicht abgewinkelte und der Zustand der Figur 6 eine abgewinkelte Gelenkposition aufzeigt.

In den Figuren 4 und 6 ist links ein Navigationssystem 10 dargestellt, das aus einem Kamerasystem, einer Rechnereinheit und zugehöriger Software besteht. Das Navigationssystem 10 kann Positionsortungen vornehmen und liefert Daten über räumliche Koordinaten von Referenzstrukturen, die es mit dem Kamerasystem erfasst und mit Hilfe dieser Daten die bildgeführte Chirurgie ermöglicht. Referenzstrukturen, die vom Navigationssystem 10 erfasst werden können, sind beispielsweise Referenzstrukturen 13 und 14, die jeweils in charakteristischer Weise angeordnete Markeranordnungen 15 und 16 aufweisen. Die Referenzstrukturen 13 und 14 sind an den Knochen 11 und 12 fixiert, hier Oberschenkelknochen 11 und Unterschenkelknochen 12. Zwischen den Gelenkfortsätzen der Knochen 11 und 12 ist die Bänderbalancevorrichtung 1 mit den Spreizplatten eingesetzt, wie sie oben beschrieben wurde.

Die Funktionsweise des erfindungsgemäßen Bänderkraft-Erfassungssystems wird nunmehr anhand einer Knieprothesen-Implantations-Operation erläutert:

Anfangs werden Femur 11 und Tibia 12 mit den Referenzanordnungen 13 und 14 versehen und im Navigationssystem 10 registriert. Je nach anteriorer Femur-Antekurvation erfolgt dann die Planung des proximalen Tibiaschnittes, eventuell mit einem Slope zwischen 5 bis 10°. Danach wird der Tibiaschnitt durchgeführt, und das Bein wird zur Streckung gebracht, wie dies in Figur 4 dargestellt ist.

Abfolgend hierauf wird die Bänderbalancevorrichtung 1 (Balancierungshilfe) in den Gelenkspalt eingebracht. Die Patella kann an ihrer anatomischen Position verbleiben, und zwar wegen der vorher schon angesprochenen bestimmten Form eines der Spreizplattenpaare. Beim Einsetzen der Vorrichtung 1 werden das mediale und das laterale Band eine Gegenkraft ausüben.

Abhängig von dem Wert dieser Kraft wird die Feder 7 (Figur 1) auf ein bestimmte Länge zusammengedrückt. Zunächst kann hierbei medial (innen) und lateral (außen) mit gleicher Kraft aufgespreizt werden, worauf sich die mechanischen Achsen der Knochen ausrichten. Aus der Differenz zwischen der unbelasteten Federlänge X (Figur 3) und der komprimierten Federlänge sowie aus der Federkonstanten lässt sich die bestehende Kraft ausrechnen, d.h. die Kraft am medialen und lateralen Band. Die Kraft kann beispielsweise an einer Skala an der Vorrichtung 1 selbst abgelesen werden.

Die Erfindung ermöglicht es, auch während der weiteren Operation die Bänderkräfte kontinuierlich festzustellen, beispielsweise dann, wenn in der Abfolge eine Bandablösung zur Korrektur der Stellung von Oberschenkel und Unterschenkel zueinander stattfindet. Dabei werden die Wegänderung im Gelenkspalt und die Federkonstante verwendet, um durchgehend Kraftdaten erhalten zu können, Die Größe des Gelenkspaltes kann mit Hilfe des Navigationssystems 10 bestimmt werden, welches ständig die Position von Femur 11 und Tibia 12 über die Referenzstrukturen 13 und 14 und die daran angebrachten Markergruppen 15 und 16 überwacht. Mit Hilfe der empfangenen Daten kann das Navigationssystem 10 die Kräfte und Spannungen der Bänder berechnen, und zwar auch bei verschiedenen Winkelstellungen des Gelenks. Deshalb erhält der Chirurg interoperativ eine Rückmeldung über die Bändersituation, die sehr hilfreich ist, um die Bänderbalancierungsprozedur und die kinematische Analyse zu unterstützen. Solche Rückmeldungen sind beispielsweise in den Screenshots der Figuren 5 und 7 dargestellt, wo nach dem Einsetzen der Vorrichtung 1 unter anderem jeweils die Werte für den Gelenkspalt angezeigt werden.

Das Navigationssystem kann auf seinem Bildschirm auch die Kraftverteilung für das mediale und laterale Band in Echtzeit anzeigen, während der Chirurg das Bein in Flexion (Beugung) bringt, nachdem die Werte für die Bandsituation bei der Streckung dem Navigationssystem gespeichert sind. Die festgestellte Bandsituation in Extension ist wichtig für die Ermittlung der Implantatposition. Zudem wird durch diese Echtzeitanzeige der in den Bändern vorherrschenden Kraft- bzw. Spannungssituation eine Bewegungsanalyse (kinematische Analyse) ermöglicht.

Eine Echtzeit-Anzeige ist erfindungsgemäß wegen der "Kommunikation" möglich, die zwischen dem Kniegelenk (Knochen mit Referenzstrukturen) der Vorrichtung 1 (bekannte Federkonstante) und dem Navigationssystem stattfindet. Mit den Werten für den Gelenkspalt bei Flexion und Extension, der Spreizkraft und der voreingestellten Federkonstante kann das Navigationssystem die Kräfte an den Bändern in verschiedene Flexionsstellungen kontinuierlich ausrechnen und ausgeben. Dabei wird die folgende einfache Formel verwendet.

$$F = k \cdot X;$$

wobei F die Kraft der Bänder, k die Federkonstante und X den Weg der, gestauchten Feder im Spreizmechanismus bezeichnet.

Im Flexionszustand wird wiederum die Balancierungsvorrichtung 1 im Gelenkspalt vorhanden sein, und die Aufspreizung erfolgt mit gleicher Bandbelastung wie bei der Extension, da die Bänder in der Extension und Flexion gleichstark gespannt sein sollen. Auch die Bandsituation in der Flexion wird vom Navigationssystem gespeichert, da die Bandsituation in Flexion wichtig für die Implantatgröße ist.

Es wird demnach eine kinematische Analyse, also eine Untersuchung der Spannungsverteilung der Bänder bei unterschiedlichen Flexionsgeraden durchgeführt, wobei das System über eine eventuelle Änderung des Gelenkspalts auf eine Änderung der Kraftsituation an den Bändern zurückrechnet. Der Chirurg weiß somit zu jedem Zeitpunkt ob zusätzliche Bandablösungen - für andere Flexionsgrade durchgeführt werden müssen.

Eine genannte kinematische Analyse kann vor- und nach der Bandablösung durchgeführt werden, wobei dann Vergleichsmöglichkeiten vorhanden sind. Wenn bei einer optimalen Bandsituation die Spannung bei unterschiedlichen Flexionswinkeln differiert, können die dann abfolgenden Knochenschnitte durch das Navigationssystem neu geplant werden, um die Situation zu verbessern. Die Kombination von Bandbalancierung, kinematischer Analyse und Resektionsplanung stellt sicher, dass eine optimale postoperative Bandsituation erzielt wird, eine optimale Planung der femoralen und tibialen Resektionsebenen stattfindet, und damit eine optimale Positionierung der Implantatkomponenten. Die "Kommunikation" zwischen der Balancierungshilfe (Hardware) und dem Navigationssystem stellt eine geeignete Ausrichtung der Beinachse und eine optimale postoperative Bandsituation in Aussicht.

Nachdem die Implantatgröße und die Schnittebene berechnet worden sind (in 90° Flexion sind posteriorer Femurschnittpunkt und proximaler Tibiaschnitt parallel) wird nunmehr die Femurresektion durchgeführt, es erfolgt eine optimale Anpassung an das Implantat, so dass nach der Resektion der Flexions- und Extensionsspalt gleich groß ist. In Abfolge hierauf wird dann die Implantation vorgenommen.

Durch die Erfindung wird es also mit einfachen Mitteln möglich, die kinematische Analyse und das Ausbalancieren der Bänder in der totalen Kniegelenkersatzoperation intraoperativ durchführen zu können, und zwar wegen des Zusammenspiels zwischen der Balancierungshilfe und dem Navigationssystem. Der Erfolg der Operation wird damit weniger von der Erfahrung des Chirurgen abhängig sondern durch die gewährte Unterstützung objektiv voraussehbarer.

**Patentansprüche**

1. Bänderkraft-Erfassungssystem mit einem Einsatz (1), der zwischen zwei durch Bänder miteinander verbundene Körperstrukturen (11, 12) einbringbar ist, und mit einer Messanordnung, welche die Kräfte bestimmt, welche auf die Bänder wirken, wobei

   - der Einsatz eine vorbestimmte Elastizität aufweist,
   - an den Körperstrukturen (11, 12) Referenzanordnungen (13, 14) angeordnet sind, und dass
   - die Messanordnung ein medizinisches Navigationssystem (10) umfasst, welches Verschiebungen der Körperstrukturen (11, 12) bzw. der Referenzstrukturen (13, 14) erfasst,

   so dass aus den Verschiebungen und der Elastizität auf die Kräfte geschlossen wird, welche auf die Bänder wirken, **dadurch gekennzeichnet, dass** der Einsatz (2) Außenkörper mit Federn und nebeneinander angeordneten Spreizfortsatzpaaren aufweist, wobei die Außenkörper (5) im Abstand verstellbar miteinander verbunden sind und wobei die Spreizfortsatzpaare hinsichtlich ihrer Länge und Abwinklung eine unterschiedliche Form besitzen.

2. System nach Anspruch 1, **dadurch gekennzeichnet, dass** die Länge der Feder und damit deren Spannung (7) durch einen Stellmechanismus veränderbar ist.

3. System nach Anspruch 1 oder 2, **dadurch gekennzeichnet, dass** in den Außenkörpern (3) des Einsatzes (1) die Federn (7) gelagert sind und das Spreizfortsatzpaar durch die Feder (7) vorgespannt ist.

4. System nach Anspruch 1, **dadurch gekennzeichnet, dass** sich die beiden Spreizfortsatzpaare im Wesentlichen parallel und im Wesentlichen in die gleiche Richtung erstrecken, insbesondere zu einer solchen Erstreckung gelenkig angeordnet sind.

5. System nach einem der Ansprüche 1 bis 4, **dadurch gekennzeichnet, dass** das Navigationssystem (10) aus den Verschiebungen und der Elastizität die Kräfte errechnet, welche auf die Bänder wirken, und diese Informationen ausgibt.

**Claims**

1. A ligament force detection system, comprising an insert (1) which can be inserted between two body structures (11, 12) connected to each other by ligaments, and comprising a measurement array which determines the forces acting on the ligaments, wherein:

- the insert exhibits a predetermined elasticity;
- reference arrays (13, 14) are arranged on the body structures (11, 12); and
- the measurement array comprises a medical navigation system (10) which detects shifts by the body structures (11, 12) and/or reference structures (13, 14),

such that the forces acting on the ligaments are deduced from the shifts and the elasticity, **characterised in that** the insert (1) comprises outer bodies with springs and pairs of stretcher extensions which are arranged adjacently, wherein the outer bodies (3) are connected to each other at an adjustable distance and wherein the pairs of stretcher extensions have a different shape with regard to their length and angle.

2. The system according to claim 1, **characterised in that** the length of the spring (7) and therefore its tension can be adjusted by an adjusting mechanism.

3. The system according to claim 1 or 2, **characterised in that** the springs (7) are mounted in the outer bodies (3) of the insert (1), and the pair of stretcher extensions are biased by the spring (7).

4. The system according to claim 1, **characterised in that** the two pairs of stretcher extensions extend substantially parallel and substantially in the same direction, in particular arranged jointed with respect to such an extension.

5. The system according to any one of claims I to 4, **characterised in that** the navigation system (10) calculates the forces acting on the ligaments from the shifts and the elasticity, and outputs this information.

**Revendications**

1. Système de détermination de force de ligaments comportant un insert (1) qui peut être introduit entre deux structures corporelles (11, 12) reliées entre elles par des ligaments, et comportant un dispositif de mesure qui détermine les forces agissant sur les ligaments, dans lequel

- l'insert présente une élasticité prédéterminée,
- des agencements de référence (13, 14) sont disposés sur les structures corporelles (11, 12), et
- le dispositif de mesure comprend un système de navigation médical (10) qui relève les déplacements des structures corporelles (1, 12) ou des structures de référence (13, 14),

ce qui fait qu'à partir des déplacements et de l'élasticité on conclut aux forces qui agissent sur les ligaments, **caractérisé en ce que** l'insert (1) comporte des corps extérieurs avec des ressorts et des paires de prolongements d'écartement disposées côte à côte, les corps extérieurs (5) étant reliés de manière que leur écartement soit variable et les paires de prolongements d'écartement présentant une forme différente quant à leur longueur et leur pliage.

2. Système selon la revendication 1, **caractérisé en ce que** la longueur des ressorts et donc leur tension (7) peut être modifiée par un mécanisme de réglage.

3. Système selon l'une quelconque des revendications 1 ou 2, **caractérisé en ce que** dans les corps extérieurs (3) de l'insert (1) sont montés les ressorts (7) et la paire de prolongements d'écartement est précontrainte par le ressort (7).

4. Système selon la revendication 1, **caractérisé en ce que** les deux paires de prolongements d'écartement s'étendent sensiblement parallèlement et sensiblement dans la même direction, et sont disposées en particulier de manière articulée pour s'étendre de la sorte.

5. Système selon l'une quelconque des revendications 1 à 4, **caractérisé en ce que** le système de navigation (10) calcule, à partir des déplacements et de l'élasticité, les forces qui agissent sur les ligaments, et délivre ces informations.

Fig.1

Fig.2

Fig.3

EP 1 645 229 B1

Fig. 4

Fig. 5

Varus

Axis Extension: 0°
Plane Flexion: 1°

1°

0°

Max: 2°

Max: 8°

Gap: 18mm

Max Ext. Gap: 19mm

7/8/02 - 10:14 AM

Fig. 6

EP 1 645 229 B1

Ext. Rotation

Axis Flexion: 34°
Plane Extension: 4°

6°

0°

Max: 12°

Max: 14°

Gap: 15mm

Max.Flex.Gap: 21mm

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5470354 A
- EP 1402857 A2
- WO 2004041097 A1
- US 4501266 A
- US 5630820 A
- DE 10221274 A1

**In der Beschreibung aufgeführte Nicht-Patentliteratur**

- A computer-assisted controlled distraction device to guide ligament balancing during knee arthroplasty. **C. MARMIGNON ; A. LEMNIEI ; P. CINQUIN ; A. HODGSON.** proceedings of the fourth annual conference of the international society for Computer assisted orthopaedic surgery caos. 19. Juni 2004